# EUROPEAN PATENT SPECIFICATION

(11) **EP 4 142 613 B1**
(45) Date of publication and mention of the grant of the patent: **04.11.2026**
(21) Application number: 21795444.5
(22) Date of filing: 30.04.2021
(51) Int. Cl.: A61B 17/04, A61B 17/00, A61B 90/00

(54) **AUTOMATIC RELEASE OF A NEAR BONE SUTURE BUTTON FROM A BUTTON INSERTER**
AUTOMATISCHE FREISETZUNG EINES KNOPFS FÜR EINE KNOCHENNAHE NAHT VON EINEM KNOPFEINFÜHRER
LIBÉRATION AUTOMATIQUE D'UN BOUTON DE SUTURE PRÈS DE L'OS À PARTIR D'UN DISPOSITIF D'INSERTION DE BOUTON

(30) Priority: 01.05.2020 US 202063018825 P
(43) Date of publication of application: 08.03.2023
(73) Proprietor: Acumed LLC, Hillsboro, OR 97124 (US)
(72) Inventor: FEDERSPIEL, Joshua P., Hillsboro, Oregon 97124 (US); SANTICH, Ann Nicole, Hillsboro, Oregon 97124 (US); VANVLEET, David William, Hillsboro, Oregon 97124 (US); HINTON, Gretchen, Hillsboro, Oregon 97124 (US); SOMMERS, Tristan, Hillsboro, Oregon 97124 (US)
(74) Representative: Boult Wade Tennant LLP
(86) International application number: PCT/US2021/030082
(87) International publication number: WO 2021/222698

(56) References cited:
- US-A1- 2009 043 318
- US-A1- 2009 043 318
- US-A1- 2017 156 717
- US-A1- 2017 303 907
- US-A1- 2018 085 110
- US-A1- 2018 085 110
- US-A1- 2018 116 659
- US-A1- 2020 100 781
- US-B2- 9 226 743

## Description

### TECHNICAL FIELD

More specifically, a button inserter and system are disclosed having a release mechanism for easily and effectively deploying a near bone button from the button inserter.

The invention is defined by the appended claims.

### BACKGROUND

Patients may sometimes suffer injuries that require securing a first, or near, bone to a second, or far, bone in order to help the patient recover from the injury. Injuries to the ankle joint may be one such type of injury. The ankle joint is composed of two bones, the tibia and fibula, which are held together by the distal tibiofibular syndesmosis. In some instances, such as after ankle injuries, the syndesmosis can be torn, leaving a gap between the tibia and fibula. Syndesmotic injuries should be repaired if found torn or unstable in order to prevent ankle instability and subsequently reduce the risk of ankle osteoarthritis.

One method to secure a near bone to a far bone is the suture button technique. The suture button technique includes deploying a bone-securing construct that includes a first (e.g., far bone) button coupled to a second (e.g., near bone) button with suture. The suture may be tensioned to secure the near bone to the far bone. For example, to repair syndesmotic injuries, the suture button technique involves two buttons that hold the fibula (e.g., near bone) and tibia (e.g., far bone) together with suture that connects the two buttons through a drilled bone hole in the fibula and tibia. The far bone button may be inserted through the drilled bone hole to secure the tibia while the near bone button secures the fibula. The buttons are typically put into place with a needle and pull-through sutures or with a button inserter.

Typical button inserters require manual removal of the near bone button (e.g., the one not inserted through the bone) from the inserter. This can be a multiple step process of loosening or unwrapping suture, and then lifting the near bone button from the handle, which can be cumbersome and time-consuming for surgeons. Accordingly, a button inserter designed to quickly and easily deploy a near bone button is desired.

Prior art document US 2009/043318 A1 discloses a technique for joint repair employing a suture/button construct and an applicator designed to securely engage a button of the suture/button construct. The button of the suture/button construct securely engages the shaft of the applicator. The suture from the button may be wrapped around a securing device (for example, a rod or bolt) located on or within the handle. The applicator with the secured button is introduced into the tissue (for example, soft tissue or a bone tunnel) and the button is passed through the tissue.

Prior art document US 2018/085110 A1 discloses a button inserter includes a shaft having a fulcrum that extends beyond a distal tip of the shaft. An inner rod is within the shaft and has a button mating surface that extends from a distal tip of the inner rod. A handle includes a handle switch that translates the inner rod between an extended position where the button mating surface of the inner rod extends beyond the distal tip of the shaft and a retracted position where the button mating surface of the inner rod is located completely within the shaft.

Prior art document US 2020/100781 A1 discloses a button inserter apparatus for the insertion of a surgical button includes an inserter handle and an elongated inserter shaft connected at one end to the inserter handle. An inserter head is connected to the inserter shaft at an end opposite to the inserter handle. The inserter head includes opposing engagement projections for engaging and retaining a surgical button there between. One of the engagement projections is longitudinally movable relative to the other of the engagement projections. A button engage and release assembly extends from the inserter handle to the inserter head, and selectively prevents relative longitudinal movement between the engagement projections, and selectively permits relative movement of the engagement projections and thereby release of the surgical button. The engage and release assembly is operable from the inserter handle to cause relative longitudinal movement of the engagement projections and release of the surgical button. A surgical button and a method of inserting a surgical button are also disclosed in this document.

### SUMMARY

The present application provides a new and innovative button inserter and system having a mechanism for deploying and releasing a near bone button from the inserter. After deploying a far bone button from the inserter, a surgeon may deploy the near bone suture button and accompanying suture in a suture button construct from the button inserter in a single motion by translating the button inserter. As such, the provided button inserter more effectively and efficiently deploys the near bone button than typical button inserters.

In light of the technical features set forth herein, and without limitation, in a first aspect, which may be combined with any other aspect unless specified otherwise, a system for suture button insertion includes a suture button construct and a suture button inserter. The suture button construct includes a far bone button joined by suture to a near bone button. The suture button inserter includes a handle, a tube, a carriage, and a spring. A portion of the handle defines a channel, a first notch, and a second notch. The tube extends from the handle. The carriage includes a body. A button post, a first suture post, and a second suture post each extend from the body. A first protrusion and a second protrusion each extend from the body. In a pre-deployment configuration of the suture button inserter, a portion of the body including the first and second protrusions is positioned within the channel of the handle such that the portion of the body may translate within the channel. A first end of the spring is in contact with a portion of the handle and a second end of the spring is in contact with the body of the carriage. Translating the carriage towards the first end of the spring compresses the spring. At least part of the portion of the body within the channel may be removed from the channel when the first and second protrusions are aligned with the first and second notches of the handle. In the pre-deployment configuration of the suture button inserter, the near bone button is positioned against the button post in the far bone button is positioned at an end of the tube such that the suture is taut between the near bone button and the far bone button.

In a second aspect, which may be combined with any other aspect unless specified otherwise, the suture is wrapped on the first suture post and the second suture post in the pre-deployment configuration of the suture button inserter.

In a third aspect, which may be combined with any other aspect unless specified otherwise, the suture is wrapped such that a first loop of the suture is formed around the first suture post, a second loop of the suture is subsequently formed around both the first suture post and the second suture post, the suture is subsequently wrapped along the outside of the second suture post, and a third loop of the suture is subsequently formed around the first suture post.

In a fourth aspect, which may be combined with any other aspect unless it's specified otherwise, the suture is wrapped on the first and second suture posts such that the suture releases from first and second suture posts in response to the near bone button being released from the carriage.

In a fifth aspect, which may be combined with any other aspect unless specified otherwise, with the far bone button deployed from the suture button inserter and maintained in a fixed position, translation of the handle of the suture button inserter away from the deployed far bone button changes a position of the carriage with respect to the channel. The change in position of the carriage enables the first and second protrusions to exit the channel through the first and second notches thereby inducing a change in orientation of the carriage which thereby releases the near bone button from the carriage.

In a sixth aspect, which may be combined with any other aspect unless specified otherwise, the change in orientation includes the carriage rotating about an axis perpendicular to the tube.

In a seventh aspect, which may be combined with any other aspect unless specified otherwise, the change in orientation of the carriage results in the first and second suture posts being positioned above the button post.

In an eighth aspect, which may be combined with any other aspect unless specified otherwise, tension in the suture between the far bone button and the near bone button maintains a position of the carriage during translation of the handle of the suture button inserter away from the far bone button.

In a ninth aspect, which may be combined with any other aspect unless specified otherwise, translation of the handle of the suture button inserter away from the far bone button causes the spring to compress between the portion of the handle and the body of the carriage.

In a tenth aspect, which may be combined with any other aspect unless specified otherwise, upon the first and second protrusions of the carriage exiting the channel through the first and second notches, the compressed spring releases energy that induces the change in orientation of the carriage.

In an eleventh aspect, which may be combined with any other aspect unless specified otherwise, a suture button inserter includes a handle, a carriage, and a spring. A portion of the handle defines a channel, a first notch, and a second notch. The carriage includes a body. A button post, a first suture post, and a second suture post each extend from the body. A first protrusion and a second protrusion each extend from the body. In a pre-deployment configuration of the suture button inserter, a portion of the body including the first and second protrusions is positioned within the channel of the handle such that the portion of the body may translate within the channel. A first end of the spring is in contact with a portion of the handle and a second end of the spring is in contact with the body of the carriage. Translating the carriage towards the first end of the spring compresses the spring. At least part of the portion of the body within the channel may be removed from the channel when the first and second protrusions are aligned with the first and second notches of the handle.

In a twelfth aspect, which may be combined with any other aspect unless specified otherwise, the button inserter further includes a tube extending from the handle, an inner rod within the tube, and an actuator for translating the inner rod.

In a thirteenth aspect, which may be combined with any other aspect unless specified otherwise, the button post includes a lip at a top end of the button post.

In a fourteenth aspect, which may be combined with any other aspect unless specified otherwise, the second end of the spring is in contact with a first end of the carriage, the first end of the carriage including the button post.
aspect unless specified otherwise, the carriage includes two separate button posts extending from the body of the carriage.

In a sixteenth aspect, which may be combined with any other aspect unless specified otherwise, the second end of the spring is in contact with a first end of the carriage, the first end of the carriage opposite a second end of the carriage, the second end of the carriage including the first and second protrusions.

In a seventeenth aspect, which may be combined with any other aspect unless specified otherwise, the second end of the spring is in contact with a first end of the carriage, the first end of the carriage opposite a second end of the carriage, the second end of the carriage including the first and second suture posts.

In an eighteenth aspect, which may be combined with any other aspect unless specified otherwise, a suture button construct includes a far bone button joined by suture to a near bone button. A method for deploying the suture button construct from a button inserter includes inserting the far bone button through a bone hole via the button inserter. The far bone button may then be deployed from the button inserter. The button inserter may then be translated away from the deployed far bone button which thereby deploys the near bone button from the button inserter.

In a nineteenth aspect, which may be combined with any other aspect unless specified otherwise, a free end of the suture is coupled to the button inserter, and translating the suture button inserter away from the deployed far bone button further deploys the free end of the suture from the button inserter.

In a twentieth aspect, which may be combined with any other aspect unless specified otherwise, the method further includes wrapping a free end of the suture around two posts of the button inserter prior to inserting the far bone button through the bone hole.

Additional features and advantages of the disclosed apparatus are described in, and will be apparent from, the following Detailed Description and the Figures. The features and advantages described herein are not all-inclusive and, in particular, many additional features and advantages will be apparent to one of ordinary skill in the art in view of the figures and description. principally selected for readability and instructional purposes, and not to limit the scope of the inventive subject matter.

### BRIEF DESCRIPTION OF THE DRAWINGS

FIG. 1 illustrates an exploded perspective view of a suture button inserter system including a suture button inserter and a suture button construct, according to an aspect of the present disclosure.
FIG. 2 illustrates a side view of the suture button inserter of FIG. 1 loaded with the suture button construct of FIG. 1 in a pre-deployment configuration, according to an aspect of the present disclosure.
FIG. 3 illustrates a top view of the suture button inserter of FIG. 1 loaded with the suture button construct of FIG. 1 in a pre-deployment configuration, according to an aspect of the present disclosure.
FIG. 4A illustrates a magnified view of the top view of FIG. 3, according to an aspect of the present disclosure.
FIG. 4B illustrates a perspective view of the carriage of the suture button inserter of FIG. 1, according to an aspect of the present disclosure.
FIGS. 5A to 5G illustrate an example suture winding method, according to an aspect of the present disclosure.
FIG. 6 illustrates a magnified view of the top view of FIG. 3 showing a deployment mechanism of the suture button inserter, according to an aspect of the present disclosure.
FIG. 7 illustrates a perspective view of the suture button inserter of FIG. 1 in a post-deployment configuration, according to an aspect of the present disclosure.
FIG. 8 illustrates a perspective view of an example far bone button, according to an aspect of the present disclosure.
FIG. 9 illustrates a perspective view of an example near bone button, according to an aspect of the present disclosure.

### DETAILED DESCRIPTION

A button inserter, system, and method are disclosed that enable surgeons, or any other suitable healthcare provider, to easily and effectively deploy a near bone button from the button inserter when implementing the suture button technique, such as to repair syndesmosis injuries in ankles. The provided button inserter includes a handle defining a channel. The handle may also define a pair of notches at an opening of the channel. A tube may extend from the handle that is part of the far bone suture button deployment mechanism of the button inserter.

The provided button inserter additionally includes a carriage. The carriage may include a body portion and a number of posts extending from the body portion. For instance, the carriage may include one or more button posts and one or more suture posts. The one or more button posts secure a positioning of a near bone button to maintain tension in the suture prior to deployment, as described more below. The one or more suture posts secure a free end of the suture prior to deployment, as described more below. The carriage may include at least two protrusions extending from the body portion. A portion of the carriage including the protrusions may be positioned within the handle's channel prior to deployment and may translate within the channel. The protrusions help prevent this carriage portion from exiting the channel until the protrusions are aligned with the handle's notches, at which point the protrusions can be translated through the notches so that the carriage may fully or partially exit the channel.

A spring, or other suitable resilient member, is present within the handle's channel. One end of the spring is in contact with the handle while the other end of the spring is in contact with the carriage. In this way, a position of the carriage within the channel may change with respect to the handle to compress the spring, or conversely, a compressed spring may apply force to the carriage as the compressed spring releases.

A suture button construct may be loaded onto the provided button inserter for deployment in a patient. The suture button construct includes a far bone button joined by suture to a near bone button. In a pre-deployment configuration, the far bone button is positioned at an end of the button inserter's tube, the near bone button is positioned against the carriage's button post(s), and the suture between the far and near bone buttons is tensioned. The suture's free end may be wrapped around the suture posts in a way that allows the wrapped suture to automatically release from the suture posts upon deployment of the near bone button. For instance, the suture's free end may be wrapped around the suture posts according to the wrapping method described below. The provided wrapping method helps prevent knot formation in the suture as it unwinds and thereby helps eliminate the need for a surgeon to manually unwind the suture.

To deploy the suture button construct from the provided button inserter, a surgeon may transport the far bone button through a bone hole via the button inserter and actuate a mechanism for deploying the far bone button. For example, the surgeon may pull a trigger that translates a rod within the button inserter's tube to force the far bone button away from the tube and thereby deploy the far bone button. With the far bone button deployed from the button inserter and against the far bone, the surgeon may translate the button inserter's handle away from the deployed far bone button (e.g., at least partially back through the bone hole). The tension in the suture between the far and near bone buttons prevents the carriage from translating with the handle. As such, translating the handle compresses the spring and increases tension in the suture, which each exert counteracting forces on the carriage. Translating the handle also changes a position of the carriage body within the handle's channel.

Once the carriage body's protrusions are aligned with the handle's notches, the combination of the tension in the suture and the force exerted on the carriage body by the spring induces the carriage to change orientation with the protrusions moving through the notches. For example, the carriage may rotate ninety degrees from a horizontal orientation to a vertical orientation. In some instances, a surgeon may help force the protrusions through the notches. For example, the surgeon may use a thumb to push upward on the carriage's suture posts (or the suture wrapped around the suture posts) to force the protrusions through the notches. The change in orientation of the carriage releases the near bone button from the carriage. For instance, the button post(s) no longer restrains the near bone button against tension in the suture with the carriage in the vertical position. Further, the vertically positioned carriage loosens the suture tension on the near bone button thereby releasing it from the carriage. The free end of the suture also releases from the suture posts. In this way, a surgeon may deploy the near bone suture button and accompanying suture in a suture button construct from the button inserter in a single motion by translating the button inserter. The surgeon may then tension the deployed suture and secure the deployed near bone button against the near bone by tying off the suture.

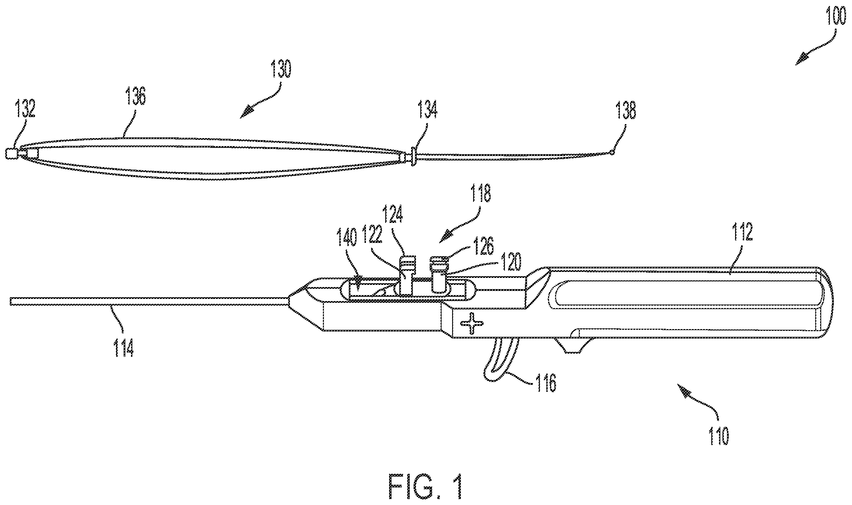

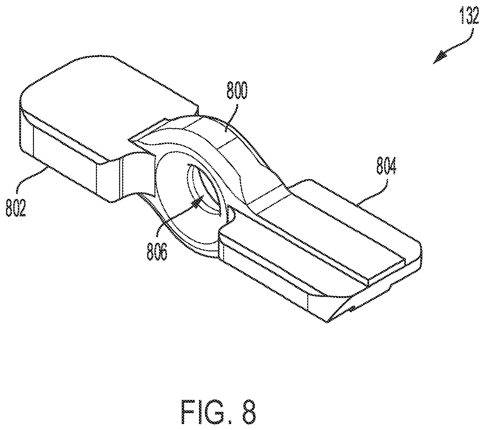

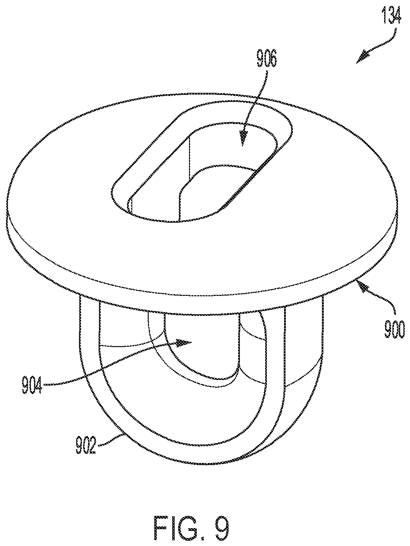

FIG. 1 illustrates an example suture button insertion system 100 including a suture button construct 130 and a button inserter 110. In at least some aspects, the suture button construct 130 includes a far bone button 132 (e.g., FIG. 8) joined by suture 136 to a near bone button 134 (e.g., FIG. 9). In some instances, a free end of the suture 136 may include a knot 138.

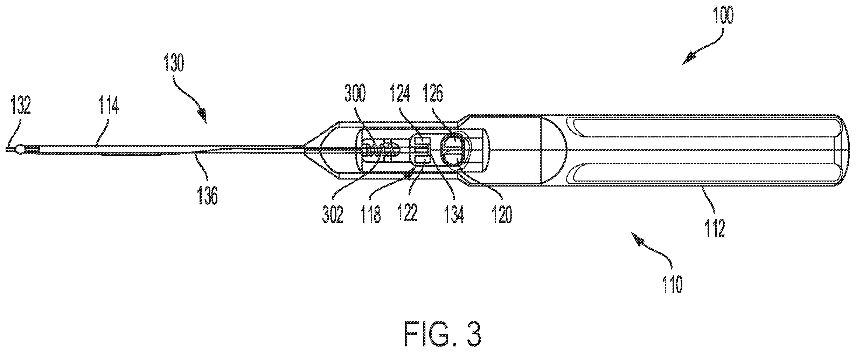

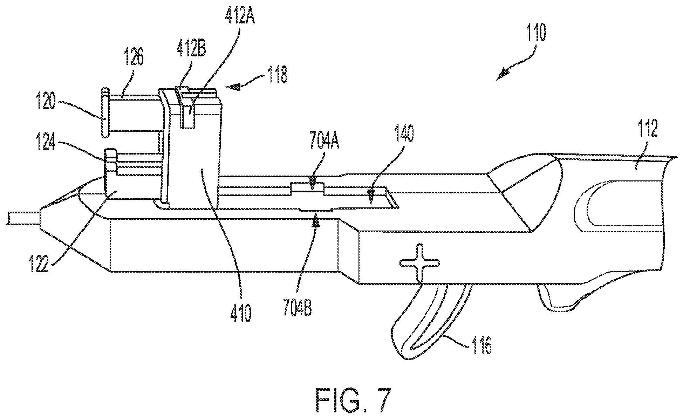

The example button inserter 110 includes a handle 112. In various aspects, a tube 114 extends from the handle 112. A rod 302 (FIG. 3) may be positioned within the tube 114. The rod 302 may be operably coupled to a trigger 116 such that actuating the trigger 116 translates the rod 302 within the tube 114. The handle 112 defines a channel 140 (best illustrated in FIG. 7). In various aspects, the handle 112 further defines at least two notches 704A and 704B (FIG. 7) at an opening of the channel 140.

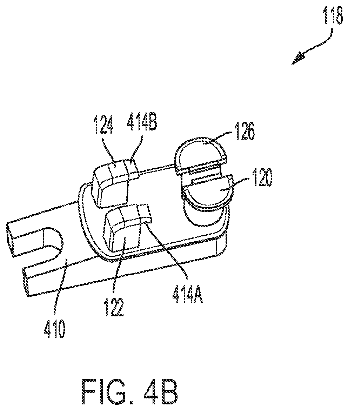

The example button inserter 110 further includes a carriage 118. FIG. 4B illustrates a perspective view of an example carriage 118. The carriage 118 may include a body 410. In various aspects, at least two protrusions 412A and 412B (FIG. 7) may extend from the body 410. A portion of the body 410 of the carriage 118 (e.g., the portion including the protrusions 412A and 412B) may be positioned within the channel 140 of the handle 112. The protrusions 412A, 412B may help prevent the portion of the body 410 of the carriage 118 from exiting the channel 140 in a pre-deployment configuration of the suture button insertion system 100. In various aspects, the carriage 118 may include one or more button posts 122, 124 extending from the body 410 of the carriage 118. In some aspects, a button post 122, 124 may include a lip 414A, 414B. While a button post 122 and a button post 124 are shown in the illustrated example, in other examples, the carriage 118 may include a single, suitable button post or more than two button posts. In various aspects, the carriage 118 may include one or more suture posts 120, 126. While a suture post 120 and a suture post 126 are shown in the illustrated example, in other examples, the carriage 118 may include a single, suitable suture post or more than two suture posts.

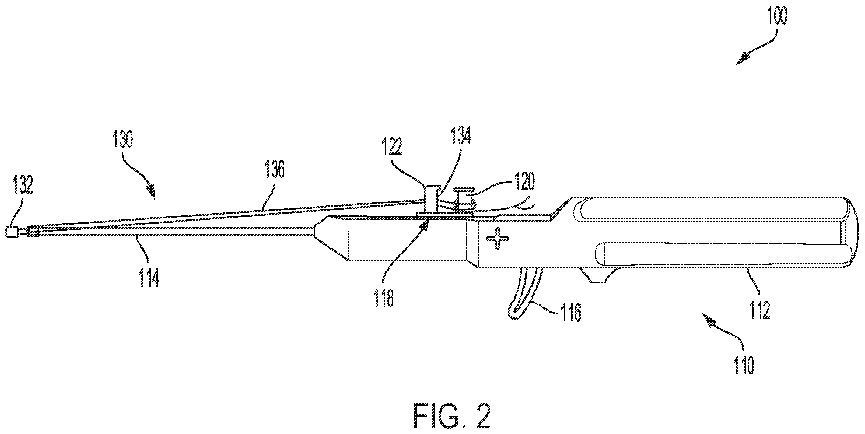

FIG. 2 illustrates a loaded, or pre-deployment configuration, of the suture button insertion system 100 with the suture button construct 130 loaded onto the button inserter 110. In the pre-deployment configuration, the far bone button 132 is positioned at an end of the tube 114. In one example, a wing 804 (FIG. 8) of the far bone button 132 may be positioned within a slit formed in the end of the tube 114. The near bone button 134 is positioned against the button posts 122 and 124. In one example, a head 900 (FIG. 9) of the near bone button 134 is positioned against the button posts 122 and 124 while a peg 902 (FIG. 9) is positioned between the button post 122 and the button post 124. In aspects in which the button posts 122, 124 have a lip 414A, 414B, the lip 414A, 414B may help prevent the near bone button 134 from translating in a direction past the lip 414A, 414B prior to deployment. The suture 136 is taut, or tensioned, between the far bone button 132 and the near bone button 134 in the pre-deployment configuration. In at least some aspects, the free end of the suture 136 may be wrapped around the button posts 122 and 124 as shown.

FIG. 3 illustrates a top view of the suture button insertion system 100 in the pre-deployment configuration. Shown in FIG. 3 is a spring 300 positioned within the channel 140 of the handle 112. One end of the spring 300 may be in contact with the handle 112 whereas the opposite end of the spring 300 may be in contact with the body 410 of the carriage 118. In various aspects, the spring 300 may surround the rod 302. While a spring 300 is utilized in the illustrated example button inserter 110, in other examples, another suitable resilient member may be substituted for the spring 300.

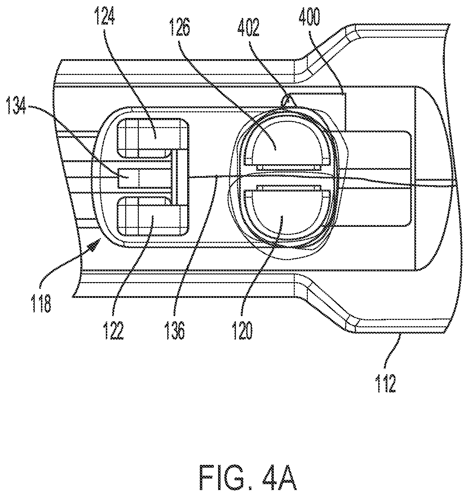

In some aspects, the button inserter 110 may include one or more indicators for determining a proper location of the carriage 118 in the pre-deployment configuration. For example, FIG. 4A illustrates a magnified top view of the button insertion system 100 showing a registration window 400 of the handle 112. In such an example, the carriage 118 may include an indicator 402 that may be aligned within the registration window 400 to position the carriage 118 at a proper location. For instance, a proper location of the carriage 118 may be when the indicator 402 is positioned at the front of the registration window 400 as illustrated. In at least some aspects, a surgeon may tension the suture 136 between the far bone button 132 in the near bone button 134 and position the carriage 118 at a proper location, and then wrap the free end of the suture 136 around the suture posts 120, 126 to maintain the position of the carriage 118. In other examples, the button inserter 110 may include another suitable indicator for determining the proper location of the carriage 118.

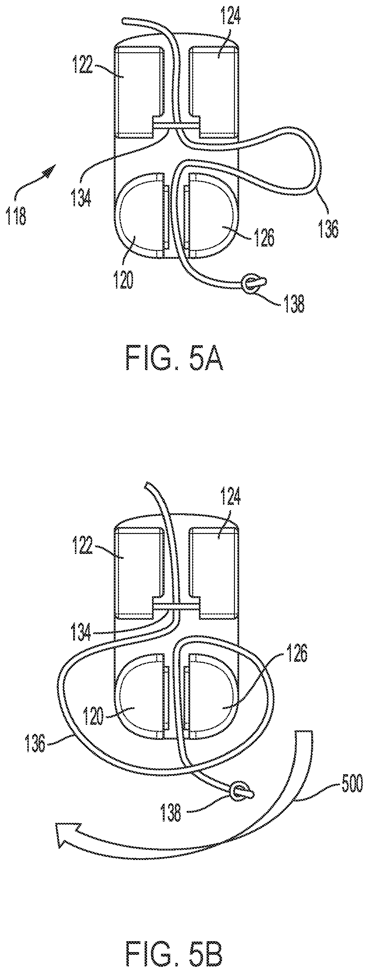

As noted, a free end of the suture 136 may be wrapped around the suture posts 120, 126 such that the suture 136 releases from the suture posts 120, 126 without forming knots and without requiring the surgeon to loosen or unwind the suture 136. FIGS. 5A to 5G illustrate an example suture wrapping method. Only a single strand of suture 136 is shown in FIGS. 5A to 5G to help more clearly and simply illustrate the various snapshots of the example suture wrapping method; however, it will be appreciated that the free end of the suture 136 may include two separate strands of suture 136 that are wrapped in unison. Stated differently, the suture 136 shown in FIGS. 5A to 5G may be two separate strands that are wrapped as shown.

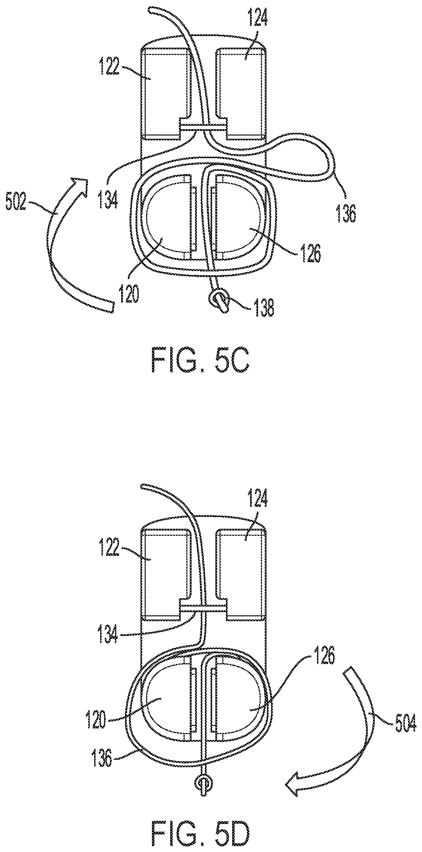

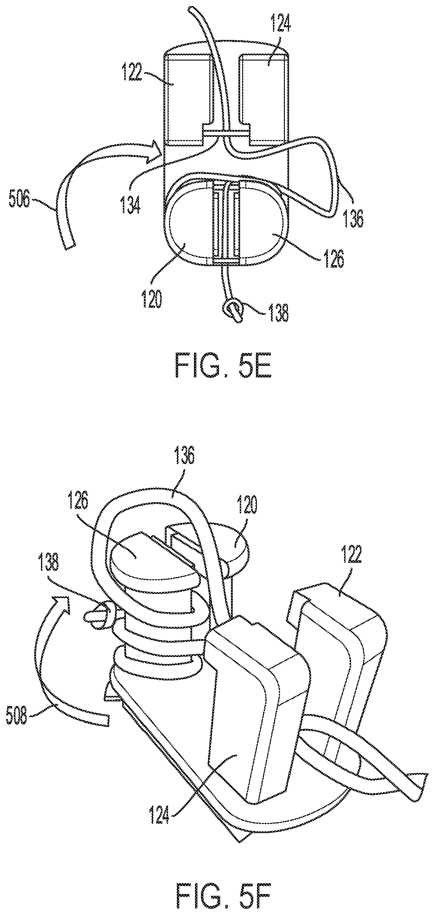

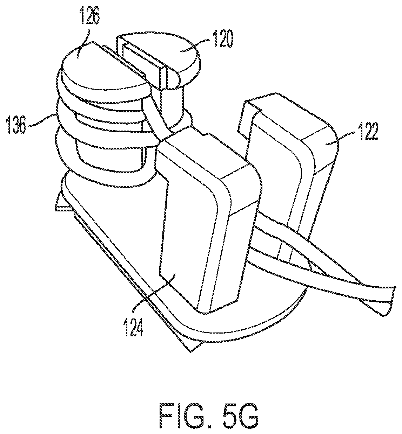

As shown in FIG. 5A, a portion of the free end of the suture 136 is first positioned between the suture post 120 and the suture post 126 while a remaining portion of the free end of the suture 136 is off to the side. As shown in FIG. 5B, the suture 136 may be wrapped in the direction of the arrow 500 thereby forming a loop of the suture 136 around the suture post 126. As shown in FIG. 5C, the suture 136 may be wrapped in the direction of the arrow 502 around the outside of the suture post 120 and then in the direction of the arrow 504 around the outside of the suture post 126, as shown in FIG. 5D, thereby forming a loop of the suture 136 around both the suture post 120 and the suture post 126. As shown in FIG. 5E, the suture 136 may then be wrapped in the direction of the arrow 506 around the outside of the suture post 120. As shown in FIG. 5F, the suture 136 may then be wrapped in the direction of the arrow 508 such that the portion of the suture 136 currently being wrapped is wrapped around the outside of the suture post 126 while the suture 136 coming from the button 134 is positioned between the suture post 120 and the suture post 126, thereby forming a loop around the suture post 126. FIG. 5G shows a final configuration of the wrapped suture 136.

A release mechanism for deploying the near bone button 134 from the example button inserter 110 will now be described. A surgeon may first transport the far bone button 132 through formed bone holes in a first and second bone using the button inserter 110. Once through the formed bone holes, the surgeon may deploy the far bone button 132 from the button inserter 110. For example, the surgeon may actuate the trigger 116 to deploy the far bone button 132. The deployed far bone button 132 positions itself against the far bone with tension in the suture 136 holding the far bone button 132 against the far bone. With the far bone button 132 deployed, the near bone button 134 may then be deployed.

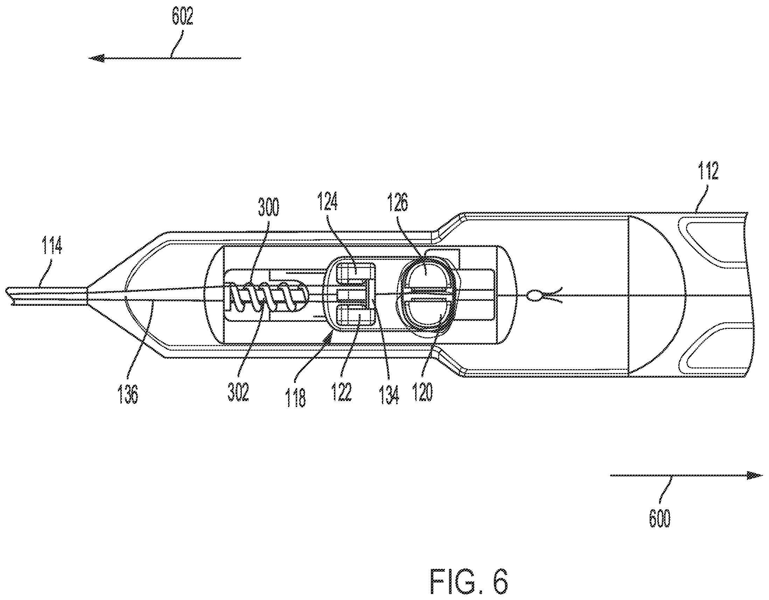

FIG. 6 illustrates a magnified top view of a portion of the suture button insertion system 100 after the far bone button 132 has been deployed from the button inserter 110. The surgeon may translate the handle 112 of the button inserter 110 away from the deployed far bone button 132 in the direction of the arrow 600. As the handle 112 is translated in the direction of the arrow 600, tension in the suture 136 prevents the carriage 118 from translating with the handle 112. Stated differently, the carriage 118 remains at least relatively stationary as the handle 112 is translated in the direction of the arrow 600 such that a position of the carriage 118 changes with respect to the channel 140 of the handle 112. The movement of the handle 112 in the direction of the arrow 600 while the carriage 118 remains stationary thereby compresses the spring 300 between the handle 112 and the carriage 118.

As the handle 112 is translated in the direction of the arrow 600 and the position of the carriage 118 changes with respect to the channel 140, a point is reached when the protrusions 412A, 412B of the carriage 118 are aligned with the notches 704A, 704B of the handle 112. At this point, a threshold amount of tension in the suture 136 forces the carriage 118 to change orientation with the protrusions 412A, 412B traveling through the notches 704A, 704B. For instance, tension in the suture 136 applies a force in the direction of the arrow 602 to the carriage 118. The force from the tension in the suture 136 at the level of the button posts 122, 124 (e.g., off-center relative to the body 410 of the carriage 118) induces rotation of the carriage 118. At the same time, an initial change in orientation of the carriage 118 induces a release of the compressed spring 300, which applies a force in the direction of the arrow 600 to the carriage 118. The counteracting force from the spring 300 (e.g., the direction of the arrow 600 is opposite the direction of the arrow 602) is in a different plane than the plane in which the force from the tension in the suture 136 resides, which thereby further induces rotation of the carriage 118.

In various aspects, the combination of the counteracting forces from the tension in the suture 136 and the spring 300 induces the carriage 118 to rotate about ninety degrees. For instance, the carriage 118 may rotate from a horizontal orientation shown in FIG. 2 to a vertical orientation shown in FIG. 7. The change in orientation of the carriage 118 releases the near bone button 134 from the carriage 118. For instance, the button posts 122, 124 no longer restrain the near bone button 134 against the tension in the suture 136 with the carriage 118 in the vertical orientation. Further, the vertical orientation of the carriage 118 loosens the tension in the suture 136 on the near bone button 134 thereby releasing the near bone button 134 from the carriage 118. Stated differently, the change in orientation of the carriage 118 in combination with the loosening tension in the suture 136 allows the near bone button 134 to separate from the button posts 122, 124 and release from the carriage 118.

As noted above, the carriage 118 may be positioned in a proper location in the pre-deployment configuration. The proper location of the carriage 118 in the pre-deployment configuration enables the counteracting forces from the tension in the suture 136 and the spring 300 to induce the change in orientation of the carriage 118 when the protrusions 702A, 702B are aligned with the notches 704A, 704B. Stated differently, in the pre-deployment configuration, the carriage 118 is positioned such that when the handle 112 is translated to deploy the near bone button 134, a threshold amount of tension is generated in the suture 136 when the protrusions 412A, 412B of the carriage 118 are aligned with the notches 704A, 704B.

As the near bone button 134 deploys from the carriage 118, and the handle 112 is further translated away from the deployed far bone button 132 (e.g., in the direction of the arrow 600), the free end of the suture 136 releases, or unwinds, from the suture posts 120, 126. The example suture wrapping method illustrated in FIGS. 5A to 5G allows the suture 136 to freely unwind from the suture posts 120 and 126 without forming a knot(s) as the near bone button 134 is deployed from the carriage 118 and the button inserter 110 is moved away from the deployed near bone button 134. FIG. 7 illustrates the button inserter 110 with the carriage 118 in a vertical orientation after deployment of the suture button construct 130.

Accordingly, a surgeon may deploy the near bone suture button 134 and accompanying suture 136 in the suture button construct 130 from the button inserter 110 in a single motion by translating the button inserter 110 away from a deployed far bone button 132. In this way, the suture button insertion system 100 helps eliminate the manual process of loosening or unwrapping the suture 136 and removing the near bone button 134 required of surgeons using typical button inserters. As such, the button inserter 110 more effectively and efficiently deploys the near bone button 134 than typical button inserters.

FIG. 8 illustrates a perspective view of an example far bone button 132. In this example, the far bone button 132 may include a base 800 integral to or connected with a wing 802 and a wing 804 that extends from the base 800. An opening 806 through which suture is routed may be defined by the base 800 of the example far bone button 132. In other examples, the far bone button 132 may have other suitable configurations.

FIG. 9 illustrates a perspective view of an example near bone button 134. In this example, the near bone button 134 may include a head 900 integral with or attached to a peg 902. The peg 902 may define an opening 904 through which suture may be routed. The head 900 includes an opening 906 through which suture may be routed. In other examples, the near bone button 134 may have other suitable configurations.

## Claims

1. A system for suture button insertion (100) comprising:
a suture button construct (130) including a far bone button (132) joined by a suture (136) to a near bone button (134); and
a suture button inserter (110) including:
a handle (112), wherein a portion of the handle defines a channel (140), a first notch (704A), and a second notch (704B);
a tube (114) extending from the handle;
a carriage (118) including a body (410), a button post (122, 124) extending from the body, a first suture post (120) and a second suture post (126) each extending from the body, and a first protrusion (412A) and a second protrusion (412B) each extending from the body, wherein a portion of the body including the first and second protrusions is positioned within the channel of the handle in a pre-deployment configuration of the suture button inserter, and wherein the portion of the body is configured to translate within the channel; and
a spring (300) having a first end in contact with a portion of the handle and a second end in contact with the body of the carriage,
wherein translating the carriage towards the first end of the spring compresses the spring,
wherein at least part of the portion of the body is configured to be removed from the channel of the handle when the first and second protrusions are aligned with the first and second notches of the handle, and
wherein in the pre-deployment configuration of the suture button inserter, the near bone button is positioned against the button post and the far bone button is positioned at an end of the tube such that the suture is taut between the near bone button and the far bone button.

2. The system for suture button insertion of claim 1, wherein the suture is wrapped on the first suture post and the second suture post in the pre-deployment configuration of the suture button inserter.

3. The system for suture button insertion of claim 2, wherein the suture is wrapped such that a first loop of the suture is formed around the first suture post, a second loop of the suture is subsequently formed around both the first suture post and the second suture post, the suture is subsequently wrapped along the outside of the second suture post, and a third loop of the suture is subsequently formed around the first suture post.

4. The system for suture button insertion of claim 2, wherein the suture is wrapped on the first and second suture posts such that the suture releases from first and second suture posts in response to the near bone button being released from the carriage.

5. The system for suture button insertion of claim 1, wherein with the far bone button deployed from the suture button inserter and maintained in a fixed position, translation of the handle of the suture button inserter away from the deployed far bone button changes a position of the carriage with respect to the channel,
wherein the change in position of the carriage enables the first and second protrusions to exit the channel through the first and second notches thereby inducing a change in orientation of the carriage which thereby releases the near bone button from the carriage.

6. The system for suture button insertion of claim 5, wherein the change in orientation includes the carriage rotating about an axis perpendicular to the tube.

7. The system for suture button insertion of claim 5, wherein the change in orientation of the carriage results in the first and second suture posts being positioned above the button post.

8. The system for suture button insertion of claim 5, wherein tension in the suture between the far bone button and the near bone button maintains a position of the carriage during translation of the handle of the suture button inserter away from the far bone button.

9. The system for suture button insertion of claim 5, wherein translation of the handle of the suture button inserter away from the far bone button causes the spring to compress between the portion of the handle and the body of the carriage.

10. The system for suture button insertion of claim 9, wherein upon the first and second protrusions of the carriage exiting the channel through the first and second notches, the compressed spring releases energy that induces the change in orientation of the carriage.

11. The system for suture button insertion of claim 1, further comprising an inner rod (302) within the tube, and an actuator (116) for translating the inner rod.

12. The system for suture button insertion of claim 1, wherein the button post includes a lip (414A, 414B) at a top end of the button post.

13. The system for suture button insertion of claim 1, wherein the second end of the spring is in contact with a first end of the carriage, the first end of the carriage including the button post.

14. The system for suture button insertion of claim 1, wherein the button post comprises two separate button posts (122, 124) extending from the body of the carriage.

15. The system for suture button insertion of claim 1, wherein the second end of the spring is in contact with a first end of the carriage, the first end of the carriage opposite a second end of the carriage, the second end of the carriage including the first and second protrusions; or
wherein the second end of the spring is in contact with a first end of the carriage, the first end of the carriage opposite a second end of the carriage, the second end of the carriage including the first and second suture posts.

## Patentansprüche

1. System zum Einsetzen von Nahtknöpfen (100), umfassend:
eine Nahtknopfkonstruktion (130), die einen fernen Knochenknopf (132) einschließt, der durch eine Naht (136) mit einem nahen Knochenknopf (134) verbunden ist; und
einen Nahtknopfeinsetzer (110), der Folgendes einschließt:
einen Griff (112), wobei ein Abschnitt des Griffs einen Kanal (140), eine erste Kerbe (704A) und eine zweite Kerbe (704B) definiert;
eine Röhre (114), die sich vom Griff aus erstreckt;
einen Schlitten (118), der einen Körper (410), einen sich vom Körper aus erstreckenden Knopfstift (122, 124), einen ersten Nahtstift (120) und einen zweiten Nahtstift (126), die sich jeweils vom Körper aus erstrecken, und einen ersten Vorsprung (412A) und einen zweiten Vorsprung (412B), die sich jeweils vom Körper aus erstrecken, einschließt, wobei ein Abschnitt des Körpers, der den ersten und den zweiten Vorsprung einschließt, in einer Vorentfaltungskonfiguration des Nahtknopfeinsetzers innerhalb des Kanals des Griffs positioniert ist und wobei der Abschnitt des Körpers dafür konfiguriert ist, sich innerhalb des Kanals zu verschieben; und
eine Feder (300), die ein erstes Ende in Kontakt mit einem Abschnitt des Griffs und ein zweites Ende in Kontakt mit dem Körper des Schlittens aufweist,
wobei das Verschieben des Schlittens in Richtung des ersten Endes der Feder die Feder zusammendrückt,
wobei zumindest ein Teil des Gehäuseabschnitts dafür konfiguriert ist, aus dem Kanal des Griffs entfernt zu werden, wenn der erste und der zweite Vorsprung mit der ersten und der zweiten Kerbe des Griffs ausgerichtet sind, und
wobei in der Vorentfaltungskonfiguration des Nahtknopfeinsetzers der nahe Knochenknopf gegen den Knopfstift positioniert ist und der ferne Knochenknopf an einem Ende der Röhre positioniert ist, so dass die Naht zwischen dem nahen Knochenknopf und dem fernen Knochenknopf straff gespannt ist.

2. System zum Einsetzen von Nahtknöpfen nach Anspruch 1, wobei die Naht in der Vorentfaltungskonfiguration des Nahtknopfeinsetzers auf dem ersten Nahtstift und dem zweiten Nahtstift aufgewickelt ist.

3. System zum Einsetzen von Nahtknöpfen nach Anspruch 2, wobei die Naht derart aufgewickelt ist, dass eine erste Schlaufe der Naht um den ersten Nahtstift gebildet ist, anschließend eine zweite Schlaufe der Naht um sowohl den ersten Nahtstift als auch den zweiten Nahtstift gebildet ist, die Naht anschließend um die Außenseite des zweiten Nahtstifts gewickelt ist und anschließend eine dritte Schlaufe der Naht um den ersten Nahtstift gebildet ist.

4. System zum Einsetzen von Nahtknöpfen nach Anspruch 2, wobei die Naht derart um den ersten und den zweiten Nahtstift gewickelt ist, dass sich die Naht als Reaktion darauf, dass der nahe Knochenknopf vom Schlitten gelöst wird, von dem ersten und dem zweiten Nahtstift löst.

5. System zum Einsetzen von Nahtknöpfen nach Anspruch 1, wobei bei aus dem Nahtknopfeinsetzer entfaltetem und in einer festen Position gehaltenem fernen Knochenknopf die Verschiebung des Griffs des Nahtknopfeinsetzers weg vom entfalteten fernen Knochenknopf eine Position des Schlittens in Bezug auf den Kanal ändert,
wobei die Änderung bei der Position des Schlittens es dem ersten und dem zweiten Vorsprung ermöglicht, den Kanal durch die erste und die zweite Kerbe zu verlassen, wodurch eine Änderung bei der Ausrichtung des Schlittens herbeigeführt wird, der dadurch den nahen Knochenknopf vom Schlitten löst.

6. System zum Einsetzen von Nahtknöpfen nach Anspruch 5, wobei die Änderung bei der Ausrichtung ein Drehen des Schlittens um eine Achse, senkrecht zur Röhre, einschließt.

7. System zum Einsetzen von Nahtknöpfen nach Anspruch 5, wobei die Änderung bei der Ausrichtung des Schlittens dazu führt, dass der erste und der zweite Nahtstift oberhalb des Knopfstifts positioniert werden.

8. System zum Einsetzen von Nahtknöpfen nach Anspruch 5, wobei die Spannung in der Naht zwischen dem fernen Knochenknopf und dem nahen Knochenknopf eine Position des Schlittens während der Verschiebung des Griffs des Nahtknopfeinsetzers weg vom fernen Knochenknopf aufrechterhält.

9. System zum Einsetzen von Nahtknöpfen nach Anspruch 5, wobei die Verschiebung des Griffs des Nahtknopfeinsetzers weg vom fernen Knochenknopf verursacht, dass sich die Feder zwischen dem Abschnitt des Griffs und dem Körper des Schlittens zusammendrückt.

10. System zum Einsetzen von Nahtknöpfen nach Anspruch 9, wobei daraufhin, dass der erste und der zweite Vorsprung des Schlittens den Kanal durch die erste und zweite Kerbe verlassen, die zusammengedrückte Feder Energie freisetzt, welche die Änderung bei der Ausrichtung des Schlittens herbeiführt.

11. System zum Einsetzen von Nahtknöpfen nach Anspruch 1, ferner umfassend eine innere Stange (302) innerhalb der Röhre und eine Betätigungsvorrichtung (116) zum Verschieben der inneren Stange.

12. System zum Einsetzen von Nahtknöpfen nach Anspruch 1, wobei der Knopfstift an einem oberen Ende des Knopfstifts eine Lippe (414A, 414B) einschließt.

13. System zum Einsetzen von Nahtknöpfen nach Anspruch 1, wobei das zweite Ende der Feder mit einem ersten Ende des Schlittens in Kontakt steht, wobei das erste Ende des Schlittens den Knopfstift einschließt.

14. System zum Einsetzen von Nahtknöpfen nach Anspruch 1, wobei der Knopfstift zwei separate Knopfstifte (122, 124) umfasst, die sich vom Körper des Schlittens aus erstrecken.

15. System zum Einsetzen von Nahtknöpfen nach Anspruch 1, wobei das zweite Ende der Feder mit einem ersten Ende des Schlittens in Kontakt steht, wobei das erste Ende des Schlittens einem zweiten Ende des Schlittens gegenüberliegt, wobei das zweite Ende des Schlittens den ersten und den zweiten Vorsprung einschließt; oder
wobei das zweite Ende der Feder mit einem ersten Ende des Schlittens in Kontakt steht, wobei das erste Ende des Schlittens einem zweiten Ende des Schlittens gegenüberliegt, wobei das zweite Ende des Schlittens den ersten und den zweiten Nahtstift einschließt.

## Revendications

1. Système d'insertion de bouton de suture (100) comprenant :
un dispositif de bouton de suture (130) incluant un bouton osseux distal (132) relié par une suture (136) à un bouton osseux proximal (134) ; et
un dispositif d'insertion de boutons de suture (110) incluant :
une poignée (112), dans lequel une partie de la poignée définit un canal (140), une première encoche (704A) et une deuxième encoche (704B) ;
un tube (114) s'étendant à partir de la poignée ;
un chariot (118) incluant un corps (410), un support de bouton (122, 124) s'étendant depuis le corps, un premier support de suture (120) et un deuxième support de suture (126) s'étendant chacun depuis le corps, et une première protubérance (412A) et une deuxième protubérance (412B) s'étendant chacune depuis le corps, dans lequel une partie du corps incluant les première et deuxième protubérances est positionnée dans le canal de la poignée dans une configuration de pré-déploiement du dispositif d'insertion de bouton de suture, et dans lequel la partie du corps est configurée pour se déplacer à l'intérieur du canal ; et
un ressort (300) présentant une première extrémité en contact avec une partie de la poignée et une seconde extrémité en contact avec le corps du chariot,
dans lequel le déplacement du chariot vers la première extrémité du ressort comprime ce dernier,
dans lequel au moins une partie du corps est configurée pour être retirée du canal de la poignée lorsque les première et deuxième protubérances sont alignées avec les première et deuxième encoches de la poignée, et
dans lequel, dans la configuration de pré-déploiement du dispositif d'insertion de bouton de suture, le bouton osseux proximal est positionné contre le support du bouton et le bouton osseux distal est positionné à une extrémité du tube de sorte que la suture soit tendue entre le bouton osseux proximal et le bouton osseux distal.

2. Système d'insertion de bouton de suture selon la revendication 1, dans lequel la suture est enroulée sur le premier et le deuxième support de suture dans la configuration de pré-déploiement du dispositif d'insertion de bouton de suture.

3. Système d'insertion de bouton de suture selon la revendication 2, dans lequel la suture est enroulée de telle sorte qu'une première boucle de suture est formée autour du premier support de suture, une deuxième boucle de suture est ensuite formée autour du premier support de suture et du deuxième support de suture, la suture est ensuite enroulée le long de l'extérieur du deuxième support de suture, et une troisième boucle de suture est ensuite formée autour du premier support de suture.

4. Système d'insertion de bouton de suture selon la revendication 2, dans lequel la suture est enroulée sur les premier et deuxième supports de suture de sorte que la suture se libère des premier et deuxième supports de suture en réponse à la libération du bouton osseux proximal du chariot.

5. Système d'insertion de bouton de suture selon la revendication 1, dans lequel, le bouton osseux distal étant déployé à partir du dispositif d'insertion de bouton de suture et maintenu dans une position fixe, la translation de la poignée du dispositif d'insertion de bouton de suture à partir du bouton osseux distal déployé modifie la position du chariot par rapport au canal,
dans lequel le changement de position du chariot permet aux première et deuxième protubérances de sortir du canal par les première et deuxième encoches, induisant ainsi un changement d'orientation du chariot qui libère le bouton osseux proximal du chariot.

6. Système d'insertion de bouton de suture selon la revendication 5, dans lequel le changement d'orientation inclut la rotation du chariot autour d'un axe perpendiculaire au tube.

7. Système d'insertion de bouton de suture selon la revendication 5, dans lequel le changement d'orientation du chariot provoque le positionnement des premier et deuxième supports de suture au-dessus du support du bouton.

8. Système d'insertion de bouton de suture selon la revendication 5, dans lequel la tension dans la suture entre le bouton osseux distal et le bouton osseux proximal maintient une position du chariot pendant la translation de la poignée du dispositif d'insertion de bouton de suture loin du bouton osseux distal.

9. Système d'insertion de bouton de suture selon la revendication 5, dans lequel la translation de la poignée du dispositif d'insertion de bouton de suture loin du bouton osseux distal provoque la compression du ressort entre la partie de la poignée et le corps du chariot.

10. Système d'insertion de bouton de suture selon la revendication 9, dans lequel, lors de la sortie du canal de la première et de la deuxième saillie du chariot à travers la première et la deuxième encoche, le ressort comprimé libère de l'énergie qui induit le changement d'orientation du chariot.

11. Système d'insertion de bouton de suture selon la revendication 1, comprenant en outre une tige intérieure (302) à l'intérieur du tube et un actionneur (116) pour déplacer la tige intérieure.

12. Système d'insertion de bouton de suture selon la revendication 1, dans lequel le support du bouton inclut un rebord (414A, 414B) à une extrémité supérieure du support du bouton.

13. Système d'insertion de bouton de suture selon la revendication 1, dans lequel la seconde extrémité du ressort est en contact avec une première extrémité du chariot, la première extrémité du chariot incluant le support du bouton.

14. Système d'insertion de bouton de suture selon la revendication 1, dans lequel le support de bouton comprend deux supports de bouton distincts (122, 124) s'étendant à partir du corps du chariot.

15. Système d'insertion de bouton de suture selon la revendication 1, dans lequel la seconde extrémité du ressort est en contact avec une première extrémité du chariot, la première extrémité du chariot étant opposée à une deuxième extrémité du chariot, la deuxième extrémité du chariot incluant les première et deuxième protubérances ; ou
dans lequel la seconde extrémité du ressort est en contact avec une première extrémité du chariot, la première extrémité du chariot opposée à une deuxième extrémité du chariot, la deuxième extrémité du chariot incluant les premier et deuxième supports de suture.
